# EUROPEAN PATENT SPECIFICATION

(11) **EP 0 184 256 B1**
(45) Date of publication and mention of the grant of the patent: **18.10.1989**
(21) Application number: 85201914.0
(22) Date of filing: 19.11.1985
(51) Int. Cl.: C07C 131/00, C07D 213/53, C07D 307/52, C07D 333/22, A01N 35/10

(54) **Diphenyl ether herbicides**
Diphenylether mit herbizider Wirkung
Diphényléthers herbicides

(30) Priority: 30.11.1984 GB 8430321
(43) Date of publication of application: 11.06.1986
(73) Proprietor: SHELL INTERNATIONALE RESEARCH MAATSCHAPPIJ B.V., 2596 HR Den Haag (NL)
(72) Inventor: Bit, Rino Antonio, Sittingbourne Kent (GB); Munro, David, Maidstone Kent (GB); Searle, Robert John Griffith, Nr. Sittingbourne Kent (GB)
(74) Representative: Bennett, David Arthur Horder

(56) References cited:
- US-A- 4 079 149

## Description

This invention relates to certain diphenyl ether derivatives, the preparation of such compounds, herbicidal composition containing them, and to their use in combating undesired plant growth.

Certain diphenyl ethers are known to be effective herbicides; for example US Patent 4419122 discloses an extensive range of 4-trifluoromethyl diphenyl ethers, and indicates that such compounds can be used as herbicides in a variety of crops. It has now been found that a group of diphenyl ethers bearing a 3-oximino substituent shows a significantly greater degree of crop selectivity than the type of diphenyl ethers specifically described in that US Patent.

Accordingly, the present invention provides 3-oximino diphenyl ether derivatives having the general formula 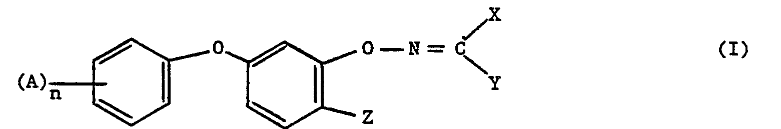 wherein n is 1, 2 or 3 and each group A, which may be the same or different when n is greater than 1, represents a halogen atom or a cyano, nitro, alkyl or haloalkyl group; Z represents a halogen atom or a nitro or cyano group; and X and Y, which may be the same or different, each independently represents a hydrogen or halogen atom, or an alkyl, cycloalkyl, haloalkyl, alkoxy, alkylthio, acyl, or optionally substituted aryl or heteroaromatic group, or one of X and Y represents one of such groups and the other represents a cyano, carboxyl, alkoxycarbonyl, phosphonic ester or alkylamino group, or X and Y together with the interjacent carbon atom may jointly represent a cycloalkyl group.

The term "acyl" is used herein to denote the radical derived from an organic acid by the removal of a hydroxyl group; the organic acid may be a carboxylic acid (including carbamic acid derivatives) or a sulphonic acid, examples of suitable acyl groups being alkylcarbonyl, alkylcarbamoyl and alkylsulphonyl, e.g. acetyl, methylsulphonyl and dimethylcarbamoyl.

When any of the foregoing substituents represents or contains an alkyl substituent group, this may be linear or branched and may contain up to 10, preferably up to 6, carbon atoms, suitable examples being methyl, ethyl, propyl and butyl. When they represent or contain a cycloalkyl substituent group this may contain from 3 to 10, preferably 5 to 8, carbon atoms, and is suitably cyclohexyl. When they contain a haloalkyl substituent group, this suitably contains up to 6, preferably up to 4, carbon atoms and the halogen atom is suitably fluorine or chlorine, trifluoromethyl being particularly preferred. When they contain an aryl substituent group, this is preferably a phenyl group. When they contain a heterocyclic group, this may, for example, be a pyrrole, pyrrolidine, pyridine, piperidine, furan, thiophene or pyran ring. When any of the foregoing substituents are designated as being optionally substituted, the substituent groups which are optionally present may be any of those customarily employed in the development of pesticidal compounds, and/or the modification of such compounds to influence their structure/activity, persistence, penetration or other property. Specific examples of such substituents include halogen, especially chlorine, atoms and nitro, cyano, alkyl or haloalkyl, especially trifluoromethyl, groups.

One substituent A is desirably located para to the ether linkage, and the other substituent(s), when present, at the position(s) ortho to the ether linkage. Preferred compounds are those wherein n is 2 or 3; one group A is a haloalkyl, especially trifluoromethyl, group which is suitably located para to the ether linkage; the second group A is a halogen, especially chlorine, atom or a nitro or cyano group which is suitably located ortho to the ether linkage; and the third group A, when present, is a halogen, especially chlorine atom.

Z preferably represents a halogen, especially bromine, atom or a nitro or cyano group.

X and Y preferably each represents a hydrogen atom or an alkyl or fluoroalkyl group of up to 6, especially up to 4, carbon atoms, such as a methyl, ethyl, propyl, butyl or trifluoromethyl group, a cyclopropyl group, an alkoxy or alkylthio group of up to 6 carbon atoms, especially a methoxy, ethoxy or methylthio group, an acyl group of formula R-CO- wherein R is a phenyl group or alkyl group of up to 6 carbon atoms, such as benzoyl, acetyl or propionyl, a phenyl, furan, thiophene or pyridyl group optionally substituted by an alkyl group such as methyl or a haloalkyl group such as trifluoromethyl, or one of X and Y represents one such group and the other represents a cyano, carboxyl or alkoxycarbonyl group of up to 6 carbon atoms, suitably a methoxycarbonyl or ethoxycarbonyl group, a dialkyl phosphonic ester group such as a diethyl phosphate, or an alkylamino group such as dimethylamino, or X and Y together with the interjacent carbon atom jointly represent a cyclopentyl or cyclohexyl group. Particularly preferred compounds are those wherein at least one of X and Y represents an alkyl group of up to 4 carbon atoms, a cylopropyl group, a trifluoromethyl group, a phenyl group or an acetyl group.

It will be appreciated that when the nature of the substituents is such as to introduce an asymmetric carbon atom, then the resulting compound will exist in sterisomeric or geometrically isomeric forms. Also, some substituent combinations permit the existence of tautomeric forms. Often, one of these isomeric forms will have greater biological activity than other forms. The scope of the present invention includes these different isomeric forms of the compounds and their mixtures, and herbicidal compositions containing the active ingredient in such isomeric forms and mixtures.

The invention also provides a process for the preparation of oximino diphenyl ethers as defined above, which comprises reacting a ketoxime of formula II 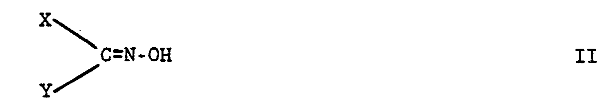 with a meta-nitro diphenyl ether of formula III 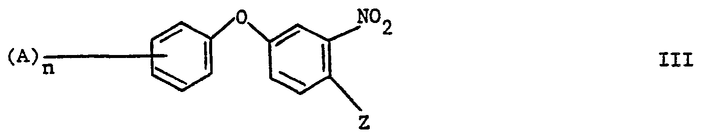 in the presence of a strong base, suitably an alkali metal hydride such as sodium hydride. The reaction is conveniently carried out in an organic solvent, which is suitably an aprotic, polar solvent such as tetrahydrofuran. The reaction may be carried out at any convenient temperature from 0°C to the boiling point of the solvent, but generally it is preferred to react under solvent reflux.

When X or Y represents an alkoxycarbonyl or phosphonic ester group, an alternative synthesis route is preferred in which the nitro phenyl ether of formula III is reacted, in the presence of a strong base, with an alkoxycarbonylhydroxylamine of formula IV; 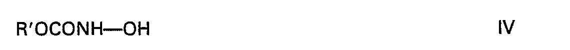 to yield a "protected" 0-phenylhydroxylamine of formula V 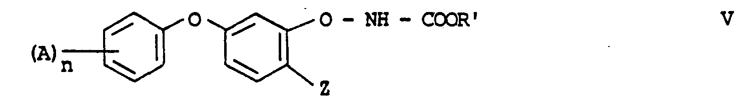 which is then deprotected, suitably by reaction with a strong organic acid such as trifluoroacetic acid, and the resulting phenylhydroxylamine is reacted with an ester of an alpha keto acid, such as ethyl pyruvate to yield the desired product wherein one of X and Y represents an alkoxycarbonyl group. Reaction of the phenylhydroxylamine with an aldehyde yields the product wherein one of X and Y represents a hydrogen atom.

The starting material of formula III may be prepared by known methods, for example as described in European Patent Application No. 2757. The final products may be isolated by any convenient recovery procedure.

The compounds of general formula I have been found to show interesting activity as herbicides. Accordingly, the invention further provides a herbicidal composition comprising a compound of formula I as defined above in association with at least one carrier, and a method of making such a composition which comprises bringing a compound of formula I into association with at least one carrier.

The invention also provides the use of such a compound or composition according to the invention as a herbicide. Further, in accordance with the invention there is provided a method of combating undesired plant growth at a locus by treating the locus with a compound or composition according to the invention. Application to the locus may be pre-emergence or post-emergence. The dosage of active ingredient used may, for example, be from 0.05 to 4 kg/ha. A carrier in a composition according to the invention is any material with which the active ingredient is formulated to facilitate application to the locus to be treated which may for example be a plant, seed or soil, or to facilitate storage, transport or handling. A carrier may be a solid or a liquid, including a material which is normally gaseous but which has been compressed to form a liquid, and any of the carriers normally used in formulating herbicidal compositions may be used. Preferably compositions according to the invention contain 0.5 to 95% by weight of active ingedient.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montmorillonites and micas; calcium carbonate, calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Agricultural compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation of fatty acids or aliphatic amines or amides containing at least 12 carbon atoms in the molecule with ethylene oxide and/or propylene oxide; fatty acid esters of glycerol, sorbitol, sucrose or pentaerythritol; condensates of these with ethylene oxide and/or propylene oxide; condensation products of fatty alcohol or alkyl phenols, for example p-octylphenol or p-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 or 75% w of active ingredient and usually contain in addition to solid inert carrier, 3-10% w of a dispersing agent and, where necessary, 0-10% w of stabiliser(s) and/or other additives such as penetrants or stickers. Dusts are usually formulated as a dust concentrate having a similar composition to that of a wettable powder but without a dispersant, and are diluted in the field with further solid carrier to give a composition usually containing 1=10% w of active ingredient. Granules are usually prepared to have a size between 10 and 100 BS mesh (1.676-0.152 mm), and may be manufactured by agglomeration or impregnation techniques. Generally, granules will contain 2 75% w active ingredient and 0-10% w of additives such as stabilisers, surfactants, slow release modifiers and binding agents. The so-called "dry flowable powders" consist of relatively small granules having a relatively high concentration of active ingredient. Emulsifiable concentrates usually contain, in addition to a solvent and, when necessary, co-solvent, 10-50% w/v active ingredient, 2-20% w/v emulsifiers and 0-20% w/v of other additives such as stabilisers, penetrants and corrosion inhibitors. Suspension concentrates are usually compounded so as to obtain a stable, non-sedimenting flowable product and usually contain 10-75% w active ingredient, 0.5-15% w of dispersing agents, 0.1-10% w of suspending agents such as protective colloids and thixotropic agents, 0-10% w of other additives such as defoamers, corrosion inhibitors, stabilisers, penetrants and stickers, and water or an organic liquid in which the active ingredient is substantialy insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the invention. The said emulsions may be of the water-in-oil or of the oil-in-water type, and may have a thick 'mayonnaise'-like consistency.

The composition of the invention may also contain other ingredients, for example other compounds possessing herbicidal, insecticidal or fungicidal properties.

The invention is illustrated in the following Examples.

### Example 1

### Preparation of O-(2-nitro-5-(2'-chloro-4'-trifluoromethyl phenoxy)phenyl) acetoxime

### A. Preparation of (2'-chloro-4'-trifluoromethyl)phenoxy-3,4-dinitrobenzene.

(i) Meta-nitrophenol (30.6 g) was dissolved in dimethylsulphoxide (100 ml), and potassium hydroxide (14.5 g) added with stirring under nitrogen at 80°C. After 1s hours, 3,4-dichlorobenzotrifluoride (43 g) was added dropwise over 30 mins and the temperature increased to 150°C before stirring overnight. Most dimethylsulphoxide was removed, diethyl ether/water (11; 50/50) added and the suspension filtered through "Celite" before separating the organic layer; this was dried and the solvent removed to give a yellow oil which was chromatographically purified (over silica with petroleum ether/chloroform eluant).
(ii) The product of step (i) - 3-chloro-4-(3'-nitrophenoxy)-benzotrifluoride - was dissolved in 1,2-dichloroethane (70 ml) and concentrated sulphuric acid (140 ml) added with stirring and cooling. When the temperature of the reaction mixture was reduced to about 2°C, potassium nitrate (11.6 g) was added portionwise over 30 minutes. The reaction mixture was then allowed to reach room temperature and stirred for 3 hours before pouring onto ice, followed by addition of sodium chloride and extraction with methylene dichloride. The organic extracts were separated, dried, and the solvent removed to give an

orange oil which was chromatographically purified and recrystallised from ethanol to yield the desired product, m.pt. 80-81°C.

### B) Preparation of title product.

Acetone oxime (2 g) was dissolved in dry tetrahydrofuran (50 ml) and dry, tetrahydrofuran washed, sodium hydride (0.8 g) added with stirring under dry nitrogen. After 1 hour a solution of the product of stage A above (9.5 g) in dry tetrahydrofuran (100 ml) was added dropwise over 15 minutes. Reaction took place rapidly. Methylene dichloride/water (400 ml; 50/50) was added and the organic solution separated and dried. Removal of solvent gave the desired product which was chromatographically purified to yield the pure title product as an orange oil. This material solidified, and was recrystallised to give pale yellow crystals, m.pt. 60-62⁰C.

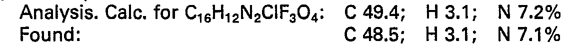

### Example 2

Preparation of O-(2-nitro-5-(2'-chloro-4'-trifluoromethyl phenoxy)phenyl cyclopentanone oxime Cyclopentanone oxime (2 g) was dissolved in dry tetrahydrofuran (50 ml) and sodium hydride (0.5 g) added portionwise with stirring under dry nitrogen. The reaction mixture was stirred for 1 hour, followed by the dropwise addition of a solution of (2'-chloro-4'-trifluoromethyl)-phenoxy-3,4-dinitrobenzene (5 g, prepared as in Example 1A) dissolved in dry tetrahydrofuran (20 ml). The reaction mixture was refluxed for 30 minutes, diethyl ether/water (500 ml, 50/50) was added, and the organic layer separated, dried and the solvent removed. Chromatographic purification followed by recrystallisation from ethyl acetate/hexane yielded the desired product, m.pt. 116-1170C.

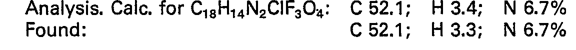

### Example 3

### Preparation of O-(2-nitro-5-(2'-chloro-4'-trifluoromethyl phenoxy)phenyl) pyruvic oxime, ethyl ester

A) A solution of 3-chloro-4-(3',4'-dinitrophenoxy)benzotrifluoride (5 g; prepared as in Example 1A) in dry tetrahydrofuran (20 ml) was added with stirring and cooling in ice to a solution of N-tert butoxycarbonyl hydroxylamine (1.8 g) in dry tetrahydrofuran (35 ml) containing sodium hydride (0.35 g). The reaction mixture was stirred at room temperature for 1 hour, then poured into diethyl ether/water (500 ml; 50.50). The organic layer was separated, dried and evaporated to leave a yellow oil which was chromatographically purified and recrystallised from ethyl acetate/petroleum ether.
B) The product of step A (2.6 g) was dissolved in trifluoroacetic acid (10 ml) and allowed to stand at room temperature for 30 minutes. The reaction mixture was then poured into water, neutralised with sodium bicarbonate and extracted with diethyl ether. The organic extracts were dried and evaporated to leave a dark red oil which was chromatographically purified.
C) Ethyl pyruvate (0.85 g) was added to a stirred solution of the product of B (2.5 g) dissolved in chloroform (100 ml) and warmed to about 50°C for half an hour. The solvent was removed on a rotary evaporator and the residue chromatographically purified to yield the E and Z geometric isomers of the desired product. Recrystallization of the E isomer from hexane and the Z isomer from ethyl acetate/hexane yielded crystalline material; m.pt of E isomer 68-70°C; m.pt for Z isomer 85-86°C.

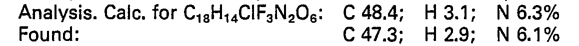

### Example 4-30

Following procedures similar to those described in the foregoing Examples, additional examples of compounds of the invention were prepared, whose melting points and chemical analyses are given in Table I below. In that Table, the compounds are identified by reference to the identity of the substituents in the following formula:

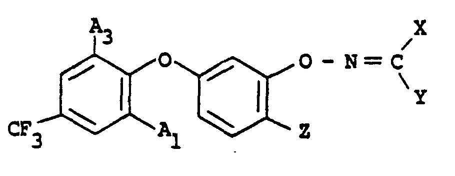

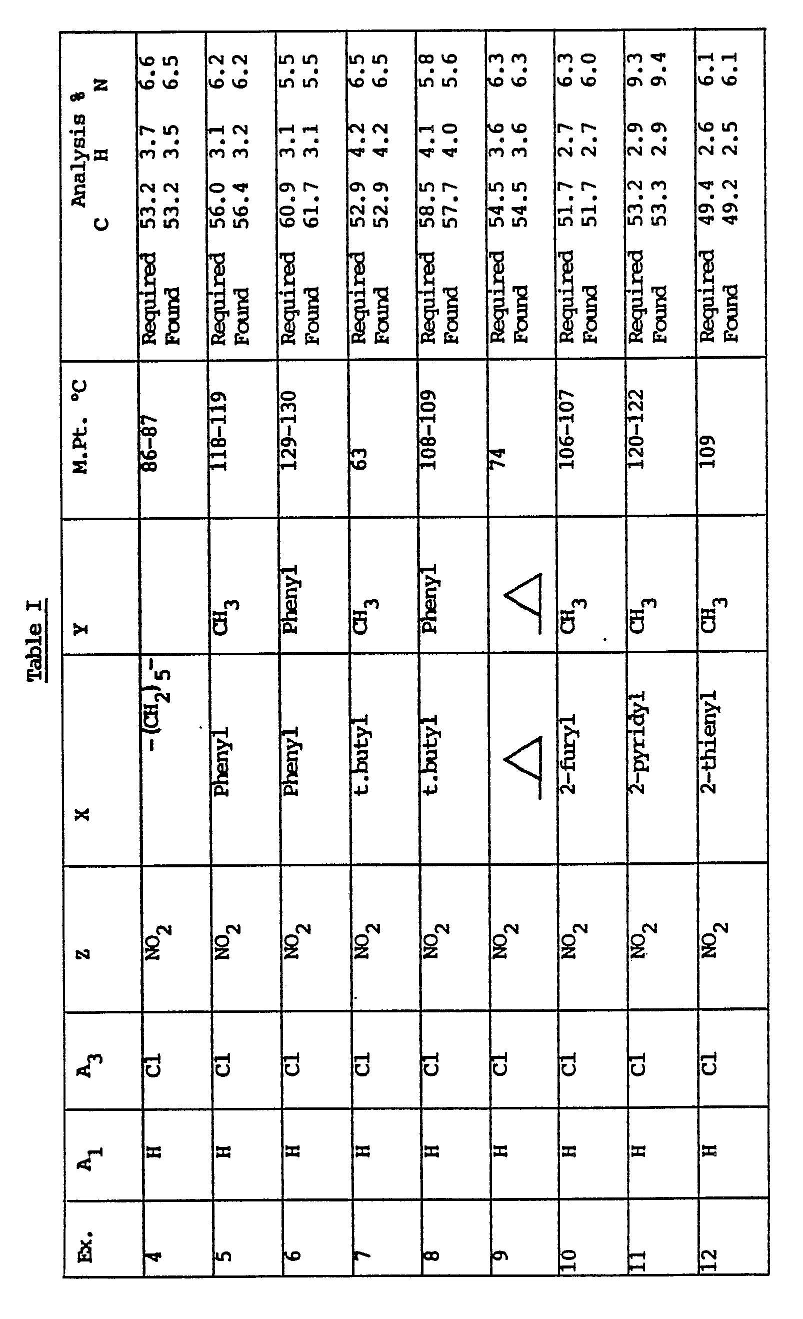

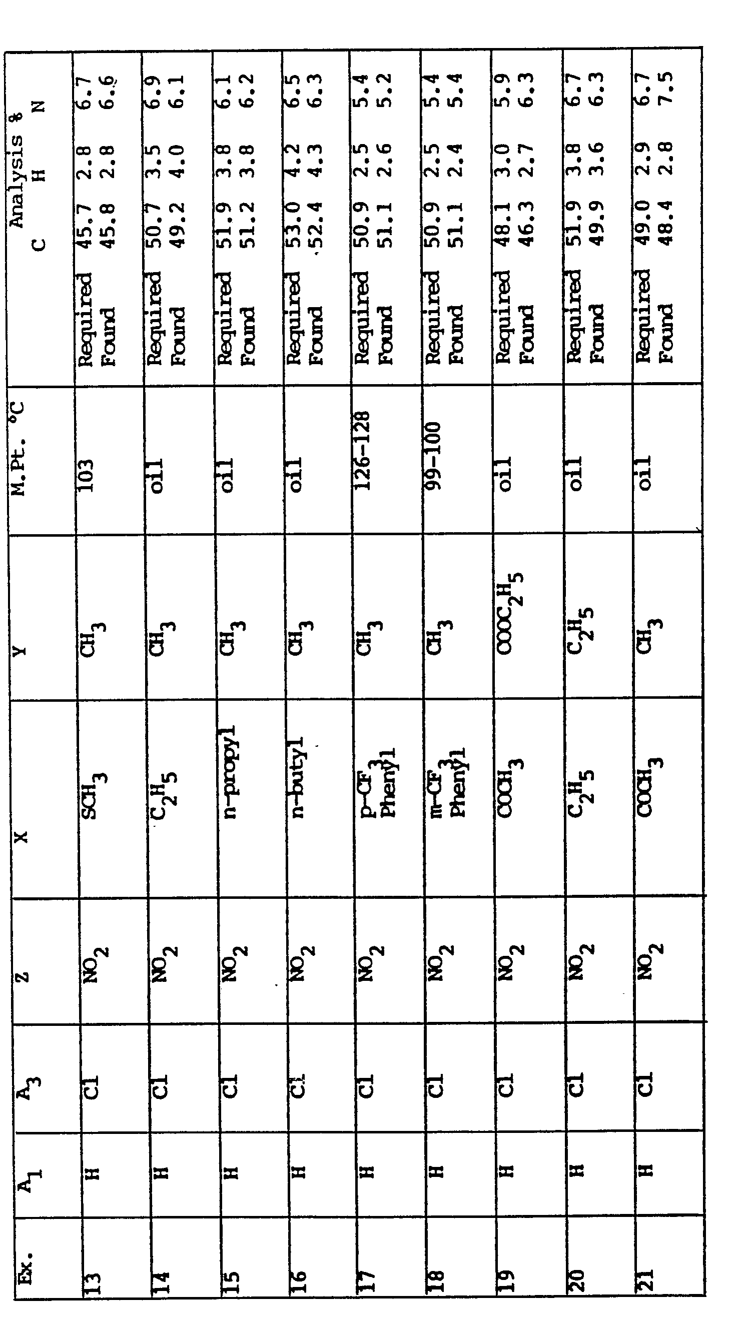

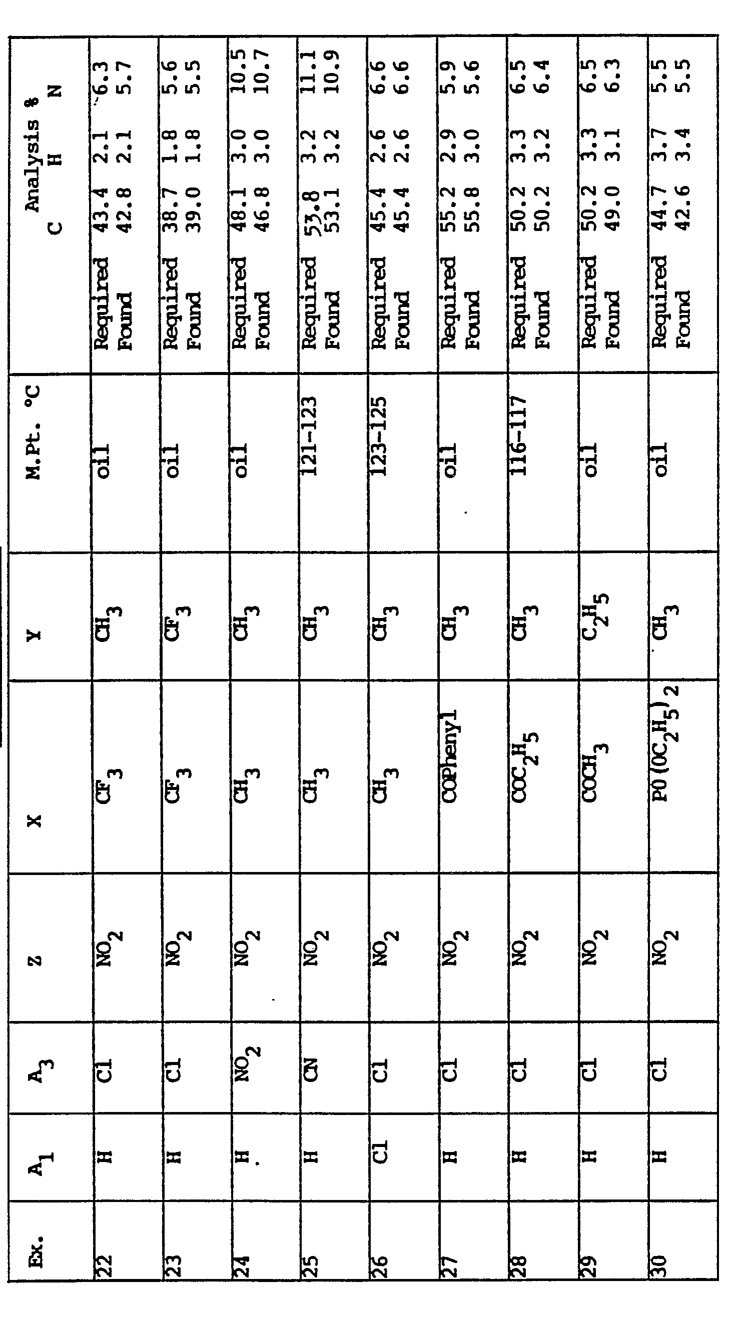

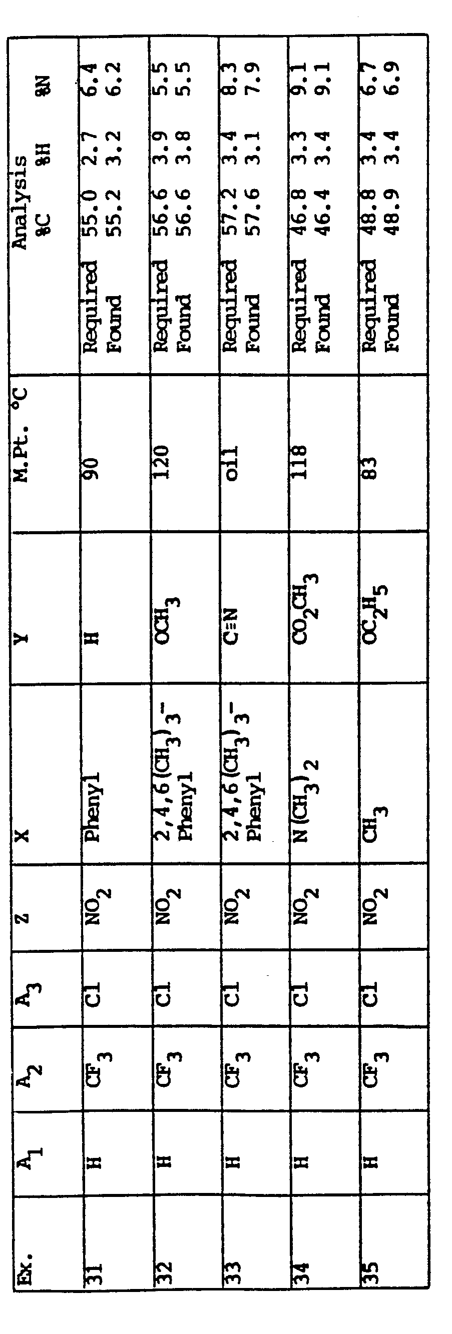

### Example 36

### Herbicidal Activity

To evaluate their herbicidal activity, compounds according to the invention were tested using as a representative range of plants: maize, Zea mays (Mz); rice, Oryza sativa (R); barnyard grass, Echinochloa crusgalli (BG); oat, Avena sativa (0); linseed, Linum usitatissisum (L); mustard, Sinapsis alba (M); sugar beet, Beta vulgaris (SB) and soya bean, Glycine max (S).

The tests fall into two categories, pre-emergence and post-emergence. The pre-emergence tests involved spraying a liquid formulation of the compound onto the soil in which the seeds of the plant species mentioned above had recently been sown. The post-emergence tests involved two types of test, viz., soil drench and foliar spray tests. In the soil drench tests the soil in which the seedling plants of the above species were growing was drenched with a liquid formulation containing a compound of the invention, and in the foliar spray tests the seedling plants were sprayed with such a formulation.

The soil used in the tests was a prepared horticultural loam.

The formulations used in the tests were prepared from solutions of the test compounds in acetone containing 0.4% by weight of an alkylphenol/ethylene oxide condensate available under the trade mark TRITON X-155. These acetone solutions were diluted with water and the resulting formulations applied at dosage levels corresponding to 5 kg or 1 kg of active material per hectare in a volume equivalent to 600 litres per hectare in the soil spray and foliar spray tests, and at a dosage level equivalent to 10 kilograms of active material per hectare in a volume equivalent to appoximately 3,000 litres per hectare in the soil drench tests.

In the pre-emergence tests untreated sown soil and in the post-emergence tests untreated soil bearing seedling plants were used as controls.

The herbicidal effects of the test compounds were assessed visually twelve days after spraying the foliage and the soil, and thirteen days after drenching the soil and were recorded on a 0-9 scale. A rating 0 indicates growth as untreated control, a rating 9 indicates death. An increase of 1 unit on the linear scale approximates to a 10% increase in the level of effect.

The results of the tests are set out in Table II below.

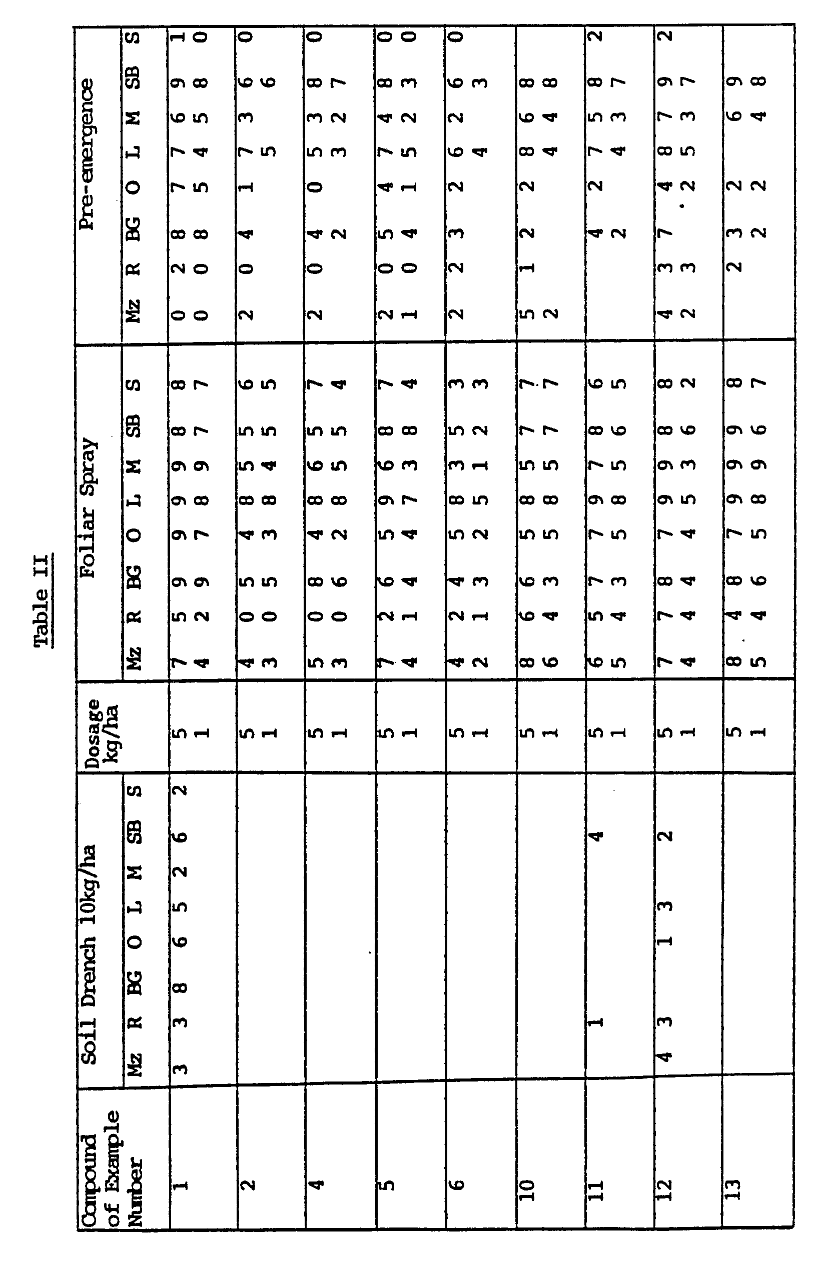

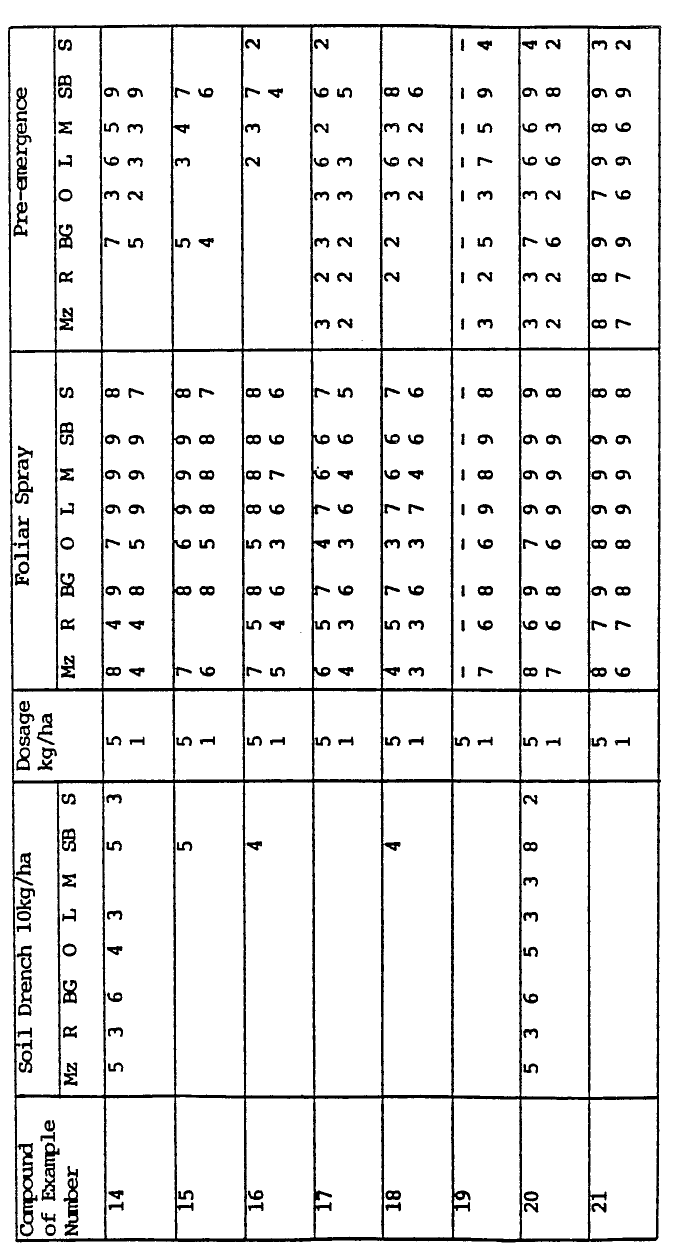

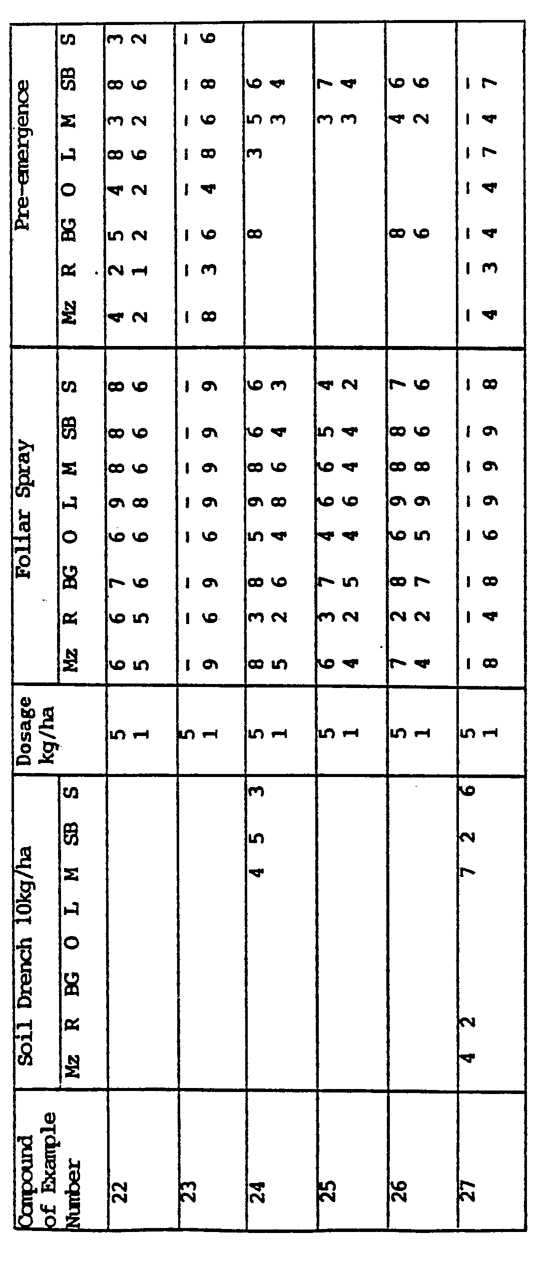

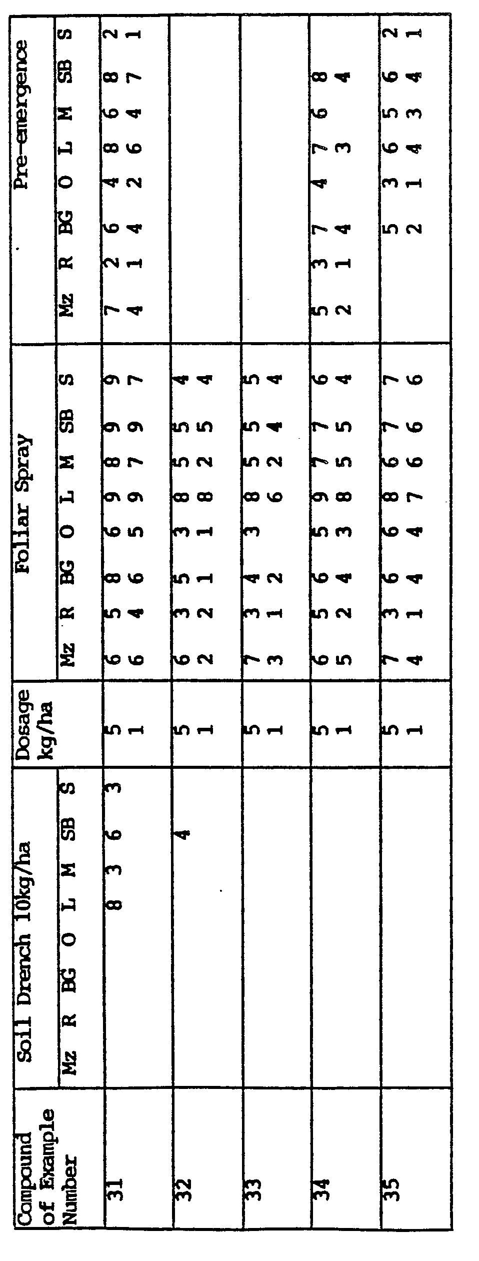

### Example 37

### Herbicidal Selectivity

A further series of biological evaluations were carried out to demonstrate the selectivity of the compounds of the invention, and to compare that selectivity with a representative selection of the diphenyl ethers specifically described in US Patent 4419122. The compounds tested were those of Examples 19, 23 and 27 hereof, and for comparison the compounds designated as numbers 3, 34 and 36 of said U.S. Patent; these three comparison compounds being designated hereafter as, respectively, A, B, and C.

The test conducted was a spray test, in which seedling plants of a range of weeds and crops were sprayed with the test compounds. The test plant species were wheat (WH) 1 true leaf and 2 true leaves, barley (BA) 1 true leaf, rice (RI) 1 true leaf and 2 true leaves, speedwell (SW) 4 true leaves, spurry (SY) 2 true leaves, black nightshade (NB) two true leaves, purslane (PR) 4 true leaves, field pansy (FP) 5 true leaves, cleavers (GG) 1 true whorl and pigweed (PI) 4 true leaves.

The soil used in the test was a prepared horticultural loan.

The compounds were tested as technical materials and formulated in a 1:1 acetone: water mix containing up to 0.2% of the wetting agent, Triton X155 and applied as single dose foliar sprays in a total volume of 600 litres/hectare. Application was at 4 different dosage levels 0.3, 0.1, 0.03 and 0.01 kg/ha designed to produce a range of responses. Three replicate pots were used for each treatment. Untreated seedling plants were used as controls.

Phytotoxicity compared with the untreated control was assessed visually using the standard 0-9 scale, 0 indicating no effect and 9 indicating death 19 days after treatment.

The results were subjected to a standard probit analysis by computer to calculate the dosage of each compound in kg/ha, required to kill 90% of the weed species and to produce 50% level of effect on the crop species. These dosages are referred to as the GIDgₒ and GIDₛₒ dosage respectively.

The GID₅₀ and GID₉₀ values obtained are set out in Table III below.

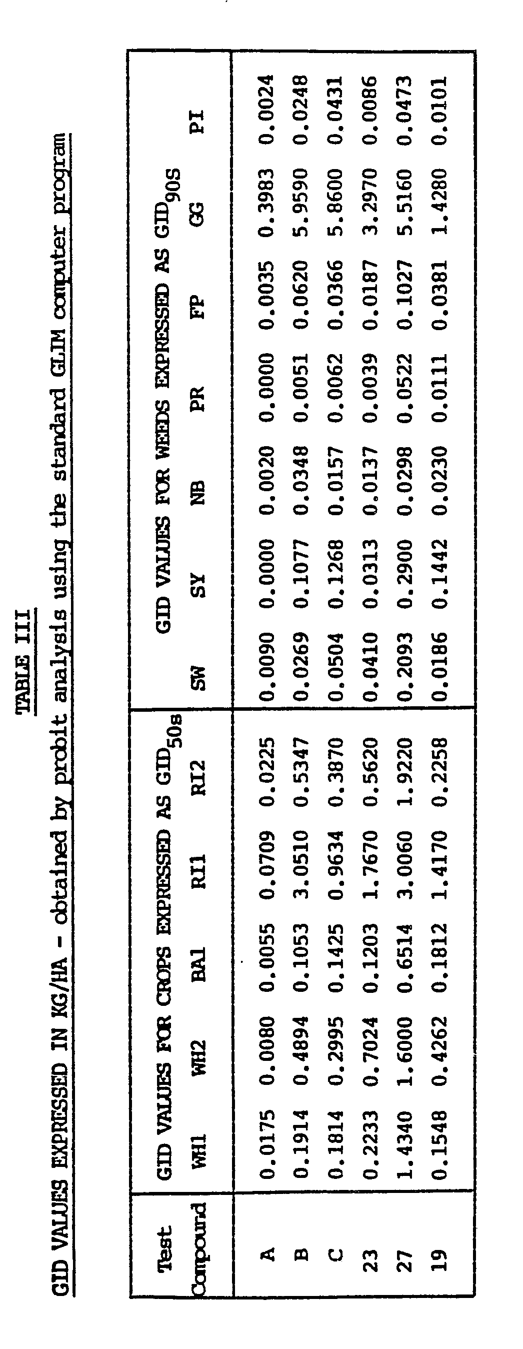

These GIDₛₒ and GID₉₀ were then used to calculate the selectivity factors for each crop by dividing the GID₅₀ of the crop by the GID₉₀ of each weed species (NB. numbers 1.0 indicate selectivity between the crop and weed and the greater the value the better the selectivity). To further facilitate comparisons between compounds the data were then standardised to Compound C using the formula:- 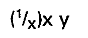 where
X = selectivity value for Compound C for a species and
y = selectivity value for a compound for that species.
The results are set out in Tables IV-VIII below.

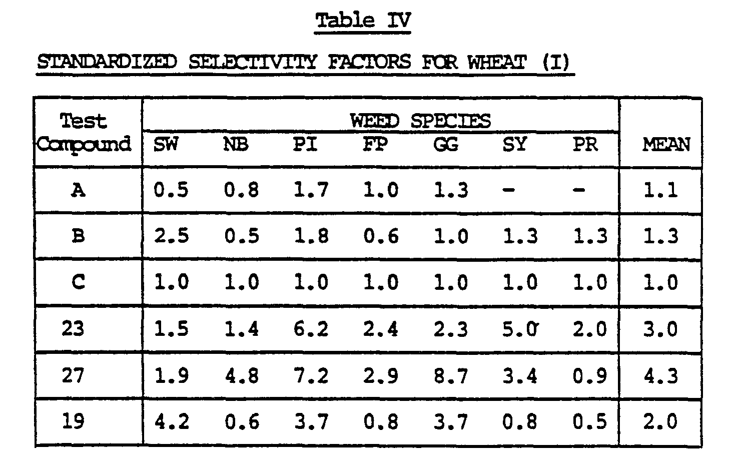

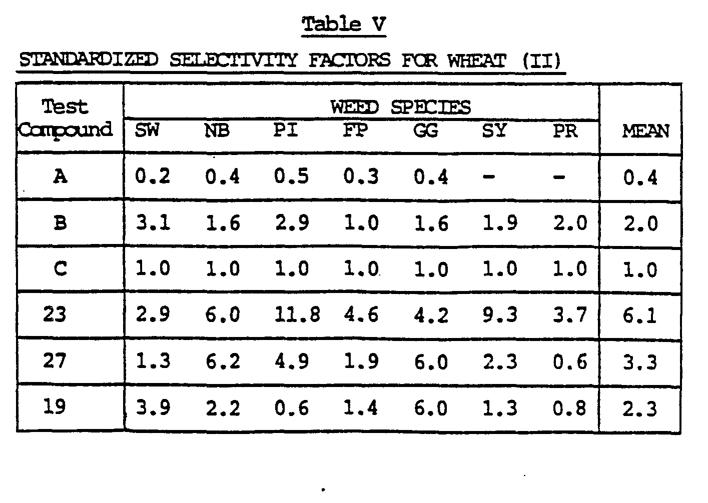

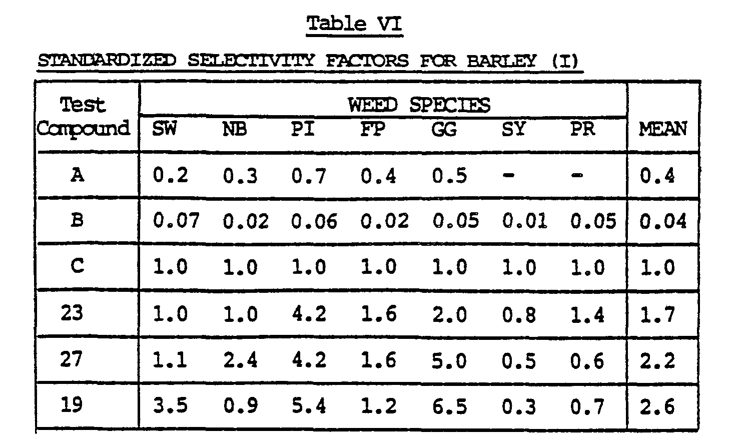

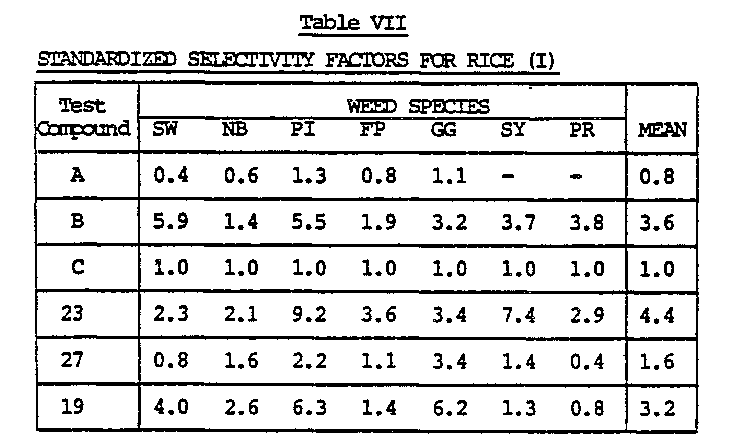

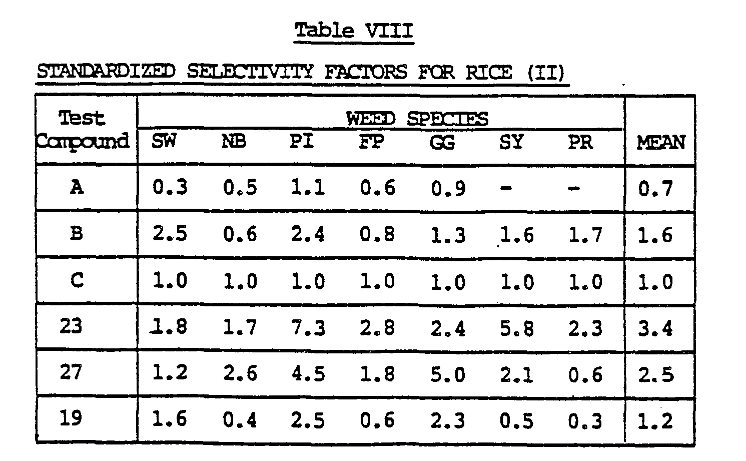

## Claims (Claims for the following Contracting State(s) : s: BE CH DE FR GB IT LI LU NL SE)

1. 3-oximino diphenyl ether derivatives having the general formula:- 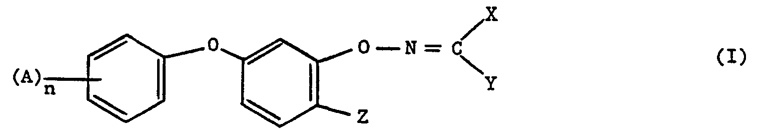 wherein n is 1, 2 or 3 and each group A, which may be the same or different when n is greater than 1, represents a halogen atom or a cyano, nitro, alkyl or haloalkyl group; Z represents a halogen atom or a nitro or cyano group; and X and Y, which may be the same or different, each independently represents a hydrogen or halogen atom or an alkyl, cycloalkyl, haloalkyl, alkoxy, alkylthio, acyl, or optionally substituted aryl or heteroaromatic group, or one of X and Y represents one of such groups and the other represents a cyano, carboxyl, alkoxycarbonyl, phosphonic ester or alkylamino group, or X and Y together with the interjacent carbon atom may jointly represent a cycloalkyl group.

2. Compounds as claimed in claim 1, wherein one substituent A is located para to the ether linkage and the other substituents, when present, are located ortho to the ether linkage, and when n is 2 or 3, one group A is a haloalkyl group, the second group A is a halogen atom or a nitro or cyano group, and the third group A, when present, is a halogen atom.

3. Compounds as claimed in claim 1 or 2 wherein X and Y each represents a hydrogen atom or an alkyl or fluoroalkyl group of up to 6 carbon atoms, a cyclopropyl group, an alkoxy or alkylthio group of up to 6 carbon atoms, an acyl group of formula RCO-, wherein R is a phenyl group or an alkyl group of up to 6 carbon atoms, a phenyl, furan, thiophene or pyridyl group optionally substituted by an alkyl or haloalkyl group, or one of X and Y represents one of such groups and the other represents a cyano, carboxyl or alkoxycarbonyl group of up to 6 carbon atoms, a dialkyl phosphonic ester group, or an alkylamino group, or X and Y together with the interjacent carbon atom jointly represent a cyclopentyl or cyclohexyl group.

4. Compounds as claimed in claim 3 wherein at least one of X and Y represents an alkyl group of up to 4 carbon atoms, a cyclopropyl group, a trifluoromethyl group, a phenyl group or an acetyl group.

5. Process for the preparation of a compound of the general formula I as defined in claim 1, which comprises reacting a ketoxime of formula II 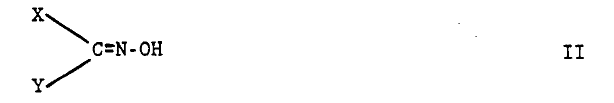 with a meta-nitro diphenyl ether of formula III 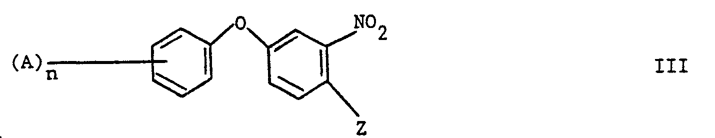 in the presence of a strong base.

6. Process as claimed in claim 5 wherein the strong base is an alkali metal hydride, and the reaction is carried out in an aprotic, polar organic solvent.

7. Herbicidal composition, which comprises a compound as claimed in any of claims 1 to 4, together with a carrier.

8. Method of combating undesired plant growth at a locus, which comprises treating the locus with a compound as claimed in any one of claims 1 to 4, or a composition as claimed in claim 7.

9. The use of a compound as claimed in any one of claims 1 to 4 as a herbicide.

## Claims (Claims for the following Contracting State(s) : AT)

1. Herbicidal composition, which comprises a compound having the general formula:- 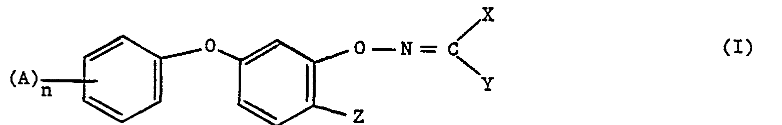 wherein n is 1, 2 or 3 and each group A, which may be the same or different when n is greater than 1, represents a halogen atom or cyano, nitro, alkyl or haloalkyl group; Z represents a halogen atom or a nitro or cyano group; and X and Y, which may be the same or different, each independently represents a hydrogen or halogen atom or an alkyl, cycloalkyl, haloalkyl, alkoxy, alkylthio, acyl, or optionally substituted aryl or heteroaromatic group, or one of X and Y represents one of such groups and the other represents a cyano, carboxyl, alkoxycarbonyl, phosphonic ester or alkylamino group, or X and Y together with the interjacent carbon atom may jointly represent a cycloalkyl group, together with a carrier.

2. A composition as claimed in claim 1, wherein in the general formula I one substituent A is located para to the ether linkage and the other substituents, when present, are located ortho to the ether linkage, and when n is 2 or 3 one group A is a haloalkyl group, the second group A is a halogen atom or a nitro or cyano group, and the third group A, when present, is a halogen atom.

3. A composition as claimed in claim 1 or 2, wherein in the general formula I X and Y each represents a hydrogen atom or an alkyl or fluoroalkyl group of up to 6 carbon atoms, a cyclopropyl group, an alkoxy or alkylthio group of up to 6 carbon atoms, an acyl group of formula RCO-, wherein R is a phenyl group or an alkyl group of up to 6 carbon atoms, a phenyl, furan, thiophene or pyridyl group _{Q}ptionally substituted by an alkyl or haloalkyl group, or one of X and Y represents one of such groups and the other represents a cyano, carboxyl or alkoxycarbonyl group of up to 6 carbon atoms, a dialkyl phosphonic ester group, or an alkylamino group, or X and Y together with the interjacent carbon atom jointly represent a cyclopentyl or cyclohexyl group.

4. A composition as claimed in claim 3, wherein in the general formula I at least one of X and Y represents an alkyl group of up to 4 carbon atoms, a cyclopropyl group, a trifluoromethyl group, a phenyl group or an acetyl group.

5. Process for the preparation of a compound of the general formula I as defined in claim 1, which comprises reacting a ketoxime of formula II 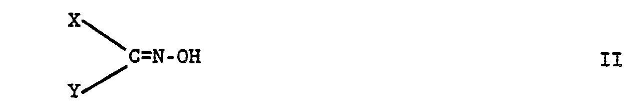 with a meta-nitro diphenyl ether of formula III 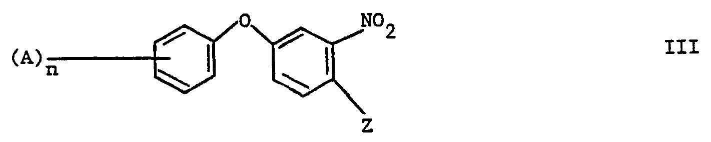 in the presence of a strong base.

6. Process as claimed in claim 5, wherein the strong base is an alkali metal hydride, and the reaction is carried out in an aprotic, polar organic solvent.

7. Method of combating undesired plant growth at a locus, which comprises treating the locus with a compound as defined in any one of claims 1 to 4, or a composition as claimed in any one of claims 1 to 4.

8. The use of a compound as defined in any one of claims 1 to 4 as a herbicide.

## Patentansprüche (Patentansprüche für folgende(n) Vertragsstaat(en) : BE CH DE FR GB IT LI LU NL SE)

1. 3-Oximino-diphenyletherderivate mit der allgemeinen Formel 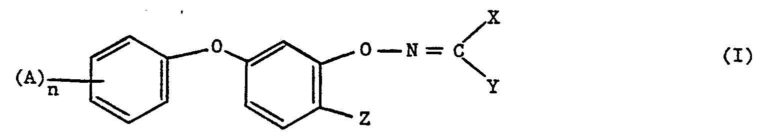 worin n den Wert 1, oder 3 aufweist und jede Gruppe A, die gleich oder verschieden sein können, wenn n größer als 1 ist, ein Halogenatom oder eine Cyano-, Nitro-, Alkyl- oder Halogenalkylgruppe bedeutet; Z ein Halogenatom oder eine Nitro- oder Cyanogruppe darstellt; und X und Y, die gleich oder verschieden sein können, jeweils unabhängig voneinander ein Wasserstoff- oder Halogenatom oder eine Alkyl-, Cycloalkyl-, Halogenalkyl-, Alkoxy-, Alkylthio-, Acyl- oder gegebenenfalls substituierte Arylgruppe oder heteroaromatische Gruppe bedeuten oder einer der Reste X und Y eine derartige Gruppe darstellt und der andere Rest eine Cyclo-, Carboxyl-, Alkoxycarbonyl-, Phosphonester- oder Alkylaminogruppe bedeutet, oder X und Y, zusammen mit dem dazwischenliegenden Kohlenstoffatom, gemeinsam eine Cykloalkylgruppe darstellen können.

2. Verbindungen nach Anspruch 1, worin ein Substituent A paraständig zur Etherverknüpfung angeordnet ist und die anderen Substituenten, soferne vorhanden, orthoständig zur Etherverknüpfung angeordnet sind, und, falls n den Wert 2 oder 3 aufweist, eine Gruppe A eine Halogenalkylgruppe bedeutet, die zweite Gruppe A ein Halogenatom oder eine Nitro- oder Cyanogruppe bedeutet und die dritte Gruppe A, soferne vorhanden, ein Halogenatom darstellt.

3. Verbindungen nach Anspruch 1 oder 2, worin X und Y jeweils ein Wasserstoffatom oder eine Alkyl-oder Fluoroalkylgruppe mit bis zu 6 Kohlenstoffatomen, eine Cyklopropylgruppe, eine Alkoxy- oder Alkylthiogruppe mit bis zu 6 Kohlenstoffatomen, eine Acylgruppe der Formel RCO-, worin R eine Phenylgruppe oder eine Alkylgruppe mit bis zu 6 Kohlenstoffatomen darstellt, oder eine Phenyl-, Furan-, Thiophen- oder Pyridylgruppe bedeuten, die gegebenenfalls durch eine Alkyl-, oder Halogenalkylgruppe substituiert ist, oder einer der Reste X und Y eine derartige Gruppe darstellt und der andere Rest eine Cyano-, Carboxyl- oder Alkoxycarbonylgruppe mit bis zu 6 Kohlenstoffatomen, eine Dialkylphosphonestergruppe oder eine Alkylaminogruppe bedeutet, oder X und Y, zusammen mit dem dazwischenliegenden Kohlenstoffatom, gemeinsam eine Cyklopentyl- oder Cyklohexylgruppe bedeuten.

4. Verbindungen nach Anspruch 3, worin höchstens einer der Reste X und Y eine Alkylgruppe mit bis zu 4 Kohlenstoffatomen, eine Cyklopropylgruppe, eine Trifluormethylgruppe, eine Phenylgruppe oder eine Acetylgruppe bedeutet.

5. Verfahren zur Herstellung einer Verbindung der allgemeinen Formel 1, wie in Anspruch 1 definiert, welches ein Umsetzen eines Ketoxims der Formel 11 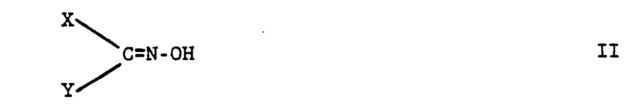 mit einem meta-Nidrodiphenylether der Formel 111 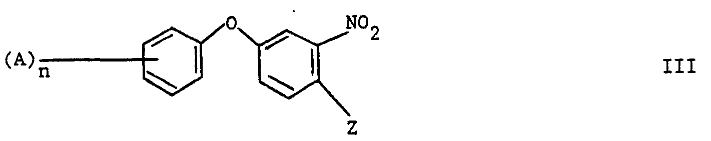 in Gegenwart einer starken Base umfaßt.

6. Verfahren nach Anspruch 5, worin die starke Base ein Alkalimetallhydrid ist und die Umsetzung in einem aprotischen, polaren organischen Lösungsmittel ausgeführt wird.

7. Herbizide Zusammensetzung, welche eine Verbindung, wie in einem der Ansprüche 1 bis 4 beansprucht, zusammen mit einem Träger umfaßt.

8. Verfahren zur Bekämpfung von unerwünschtem Pflanzenwuchs an einem Ort, welches ein Behandeln des Ortes mit einer Verbindung, wie in einem der Ansprüche 1 bis 4 beansprucht, oder mit einer Zusammensetzung, wie in Anspruch 7 beansprucht, umfaßt.

9. Verwendung einer Verbindung, wie in einem der Ansprüche 1 bis 4 beansprucht, als ein Herbizid.

## Patentansprüche (Patentansprüche für folgende(n) Vertragsstaat(en) : AT)

1. Herbizide Zusammensetzung, welche eine Verbindung mit der allgemeinen Formel: 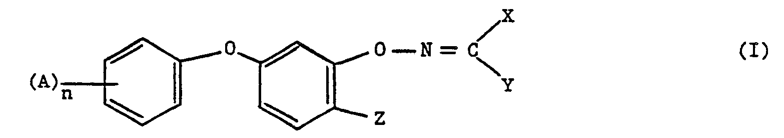 worin n den Wert 1, 2 oder 3 aufweist und jede Gruppe A, die gleich oder verschieden sein können, wenn n größer als 1 ist, ein Halogenatom oder eine Cyano-, Nitro-, Alkyl- oder Halogenalkylgruppe bedeutet; Z ein Halogenatom oder eine Nitro- oder Cyanogruppe darstellt; und X und Y, die gleich oder verschieden sein können, jeweils unabhängig voneinander ein Wasserstoff- oder Halogenatom oder eine Alkyl-, Cycloalkyl-, Halogenalkyl-, Alkoxy-, Alkylthio-, Acyl- oder gegebenenfalls substituierte Arylgruppe oder heteroaromatische Gruppe bedeuten oder einer der Reste X und Y eine derartige Gruppe darstellt und der andere Rest eine Cyano-, Carboxyl-, Alkoxycarbonyl-, Phosphonester- oder Alkylaminogruppe bedeutet, oder X und Y, zusammen mit dem dazwischenliegenden Kohlenstoffatom, gemeinsam eine Cykloalkylgruppe darstellen können, zusammen mit einem Träger umfaßt.

2. Zusammensetzung nach Anspruch 1, worin in der allgemeinen Formel I ein Substituent A paraständig zur Etherverknüpfung angeordnet ist und die anderen Substituenten, soferne vorhanden, orthoständig zur Etherverknüpfung vorliegen, und worin dann, wenn n den Wert 2 oder 3 aufweist, eine Gruppe A eine Halogenalkylgruppe bedeutet, die zweite Gruppe A ein Halogenatom oder eine Nitro- oder Cyanogruppe bedeutet und die dritte Gruppe A, soferne vorhanden, ein Halogenatom bedeutet.

3. Zusammensetzung nach Anspruch 1 oder 2, worin in der allgemeinen Formel I X und Y jeweils für ein Wasserstoffatom oder eine Alkyl- oder Fluoralkylgruppe mit bis zu 6 Kohlenstoffatomen, eine Cykloporopylgruppe, eine Alkoxy- oder Alkylthiogruppe mit bis zu 6 Kohlenstoffatomen, eine Acylgruppe der Formel RCO-, worin R eine Phenylgruppe oder eine Alkylgruppe mit bis zu 6 Kohlenstoffatomen bedeutet, oder eine Phenyl-, Furan-, Thiophen- oder Pyridylgruppe, die gegebenenfalls durch eine Alkyl-, oder Halogenalkylgruppe substituiert ist, stehen, oder einer der Reste X und Y eine derartige Gruppe darstellt und der andere Rest für eine Cyano-, Carboxyl- oder Alkoxycarbonylgruppe mit bis zu 6 Kohlenstoffatomen, eine Dialkylphosphonestergruppe oder eine Alkylaminogruppe steht, oder X und Y, zusammen mit dem dazwischenliegenden Kohlenstoffatom, gemeinsam eine Cyklopentyl- oder Cyklohexylgruppe darstellen.

4. Zusammensetzung nach Anspruch 3, worin in der allgemeinen Formel wenigstens einer der Reste X und Y eine Alkylgruppe mit bis zu 4 Kohlenstoffatomen, eine Cyklopropylgruppe, eine Trifluormethylgruppe, eine Phenylgruppe oder eine Acetylgruppe bedeutet.

5. Verfahren zur Herstellung einer Verbindung der allgemeinen Formel I, wie in Anspruch 1 definiert, welches ein Umsetzen eines Ketoxims der Formel 11 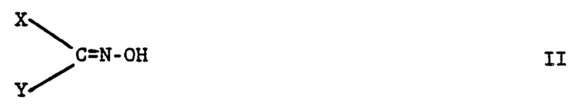 mit einem meta-Nitrodiphenylether der Formel 111 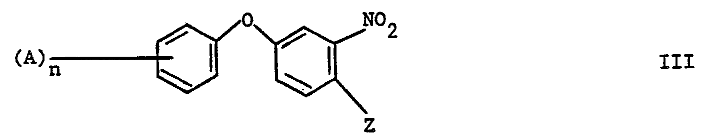 in Gegenwart einer starken Base umfaßt.

6. Verfahren nach Anspruch 5, woerin die starke Base ein Alkalimetallhydrid ist und die Umsetzung in einem aprotischen, polaren organischen Lösungsmittel ausgeführt wird.

7. Verfahren zur Bekämpfung von unerwünschtem Pflanzenwuchs an einem Ort, welches ein Behandeln des Ortes mit einer Verbindung, wie in einem der Ansprüche 1 bis 4 definiert, oder mit einer Zusammensetzung, wie in einem der Ansprüche 1 bis 4 definiert, umfaßt.

8. Verwendung einer Verbindung, wie in einem der Ansprüche 1 bis 4 definiert, als ein Herbizid.

## Revendications (Revendications pour l'(les) Etat(s) contractant(s) suivant(s) : BE CH DE FR GB IT LI LU NL SE)

1. Dérivés de 3-oximino diphényléther ayant la formule générale 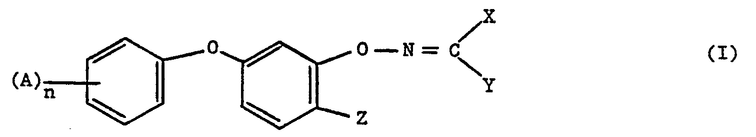 où n est 1, 2 ou 3 et chaque groupe A, ces groupes pouvant être identiques ou différents quand n est plus grand que 1, représente un atome d'halogène ou un groupe cyano, nitro, alcoyle ou haloalcoyle; Z représente un atome d'halogène ou un groupe nitro ou cyano; et X et Y, qui peuvent être identiques ou différents, représentent chacun indépendamment un atome d'hydrogène ou d'halogène ou un groupe alcoyle, cycloalcoyle, haloalcoyle, alcoxy, alcoylthio, acyle ou un groupe aryle ou hétéro-aromatique éventuellement substitué, ou un de X et Y représente un de ces groupes et l'autre représente un groupe cyano, carboxyle, alcoxycarbonyle, ester phosphonique ou alcoylamino, ou X et Y en même temps que l'atome de carbone intermédiaire peuvent représenter conjointement un groupe cycloalcoyle.

2. Composés selon la revendication 1, dans lequel un substituant A est situé en position para par rapport à la liaison éther et les autres substituants, quand ils sont présents, sont situés en position ortho par rapport à la liaison éther et, quand n est 2 ou 3, un groupe A est un groupe haloalcoyle, le deuxième groupe A est un atome d'halogène ou un groupe nitro ou cyano et le troisième groupe A, quand il est présent, est un atome d'halogène.

3. Composés selon la revendication 1 ou 2, dans lesquels X et Y représentent chacun un atome d'hydrogène ou un groupe alcoyle ou fluoroalcoyle ayant jusqu'à 6 atomes de carbone, un groupe cyclopropyle, un groupe alcoxy ou alcoylthio ayant jusqu'à 6 atomes de carbone, un groupe acyle de formule RCO-, où R est un groupe phényle ou un groupe alcoyle ayant jusqu'à 6 atomes de carbone, un groupe phényle, furanne, thiophène ou pyridyle éventuellement substitué par un groupe alcoyle ou haloalcoyle, ou un de X et Y représente un de ces groupes et l'autre représente un groupe cyano, carboxyle ou alcoxycarbonyle ayant jusqu'à 6 atomes de carbone, un groupe ester dialcoyl phosphonique ou un groupe alcoylamino, ou X et Y en même temps que l'atome de carbone intermédiaire représentent conjointement un groupe cyclopentyle ou cyclohexyle.

4. Composés selon la revendication 3, dans lesquels au moins un de X et Y représente un groupe alcoyle ayant jusqu'à 4 atomes de carbone, un groupe cyclopropyle, un groupe trifluorométhyle, un groupe phényle ou un groupe acétyle.

5. Procédé de préparation d'un composé de la formule générale 1 tel que défini dans la revendication 1, qui comprend la réaction d'une cétoxime de formule Il 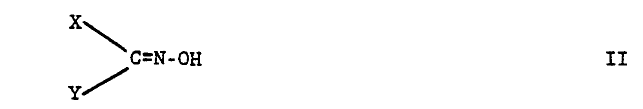 avec un méta-nitro diphényléther de formule III 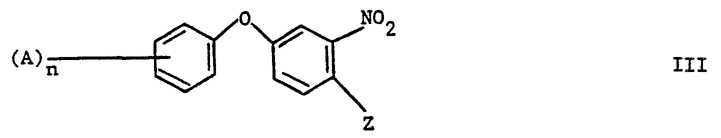 en présence d'une base forte.

6. Procédé selon la revendication 5, dans lequel la base forte est un hydrure de métal alcalin et la réaction est conduite dans un solvant organique aprotique polaire.

7. Composition herbicide, qui comprend un composé selon l'une quelconque des revendications 1 à 4 en même temps qu'un véhicule.

8. Procédé de lutte contre la croissance de plantes indésirables en un lieu, qui comprend le traitement du lieu par un composé selon l'une quelconque des revendications 1 à 4 ou une composition selon la revendication 7.

.9. L'utilisation d'un composé selon l'une quelconque des revendications 1 à 4 comme herbicide.

## Revendications (Revendications pour l'(les) Etat(s) contractant(s) suivant(s) : AT)

1. Composition herbicide, qui comprend un composé ayant la formule générale: 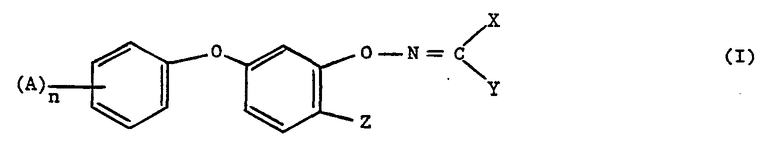 où n est 1, 2 ou 3 et chaque groupe A, ces groupes pouvant être identiques ou différents quand n est plus grand que 1, représente un atome d'halogène ou un groupe cyano, nitro, alcoyle ou haloalcoyle; Z représente un atome d'halogène ou un groupe nitro ou cyano; et X et Y, qui peuvent être identiques ou différents, représentent chacun indépendamment un atome d'hydrogène ou d'halogène ou un groupe alcoyle, cycloalcoyle, haloalcoyle, alcoxy, alcoylthio, acyle ou un groupe aryle ou hétéro-aromatique éventuellement substitué, ou un de X et Y représente un de ces groupes et l'autre représente un groupe cyano, carboxyle, alcoxycarbonyle, ester phosphonique ou alcoylamino, ou X et Y en même temps que l'atome de carbone intermédiaire représentent conjointement un groupe cycloalcoyle, en même temps q'une véhicule.

2. Une composition selon la revendication 1, dans laquelle dans la formule générale 1 un substituant A est situé en position para par rapport à la liaison éther, et les autres substituants, quand ils sont présents, sont situés en position ortho par rapport à la liaison éther, et quand n est 2 ou 3, un groupe A est un groupe haloalcoyle, le deuxième groupe A est un atome d'halogène ou un groupe nitro ou cyano et le troisième groupe A, quand il est présent, est un atome d'halogène.

3. Une composition selon la revendication 1 ou 2, dans laquelle dans la formule générale I X et Y représentent chacun un atome d'hydrogène ou un groupe alcoyle ou fluoroalcoyle ayant jusqu'à 6 atomes de carbone, un groupe cyclopropyle, un groupe alcoxy ou alcoylthio ayant jusqu'à 6 atomes de carbone, un groupe acyle de formule RCO-, où R est un groupe phényle ou un groupe alcoyle ayant jusqu'à 6 atomes de carbone, un groupe phényle, furanne, thiophène ou pyridyle éventuellement substitué par un groupe alcoyle ou haloalcoyle, ou un de X et Y représente un de ces groupes et l'autre représente un groupe cyano, carboxyle ou alcoxycarbonyle ayant jusqu'à 6 atomes de carbone, un groupe ester dialcoyl phosphonique ou un groupe alcoylamino, ou X et Y en même temps que l'atome de carbone intermédiaire représentent conjointement un groupe cyclopentyle ou cyclohexyle.

4. Une composition selon la revendication 3, dans laquelle dans la formule générale 1 au moins un de X et Y représente un groupe alcoyle ayant jusqu'à 4 atomes de carbone, un groupe cyclopropyle, un groupe trifluorométhyle, un groupe phényle ou un groupe acétyle.

5. Procédé de préparation d'un composé de la formule générale 1 tel que défini dans la revendication 1, qui comprend la réaction d'une cétoxime de formule Il 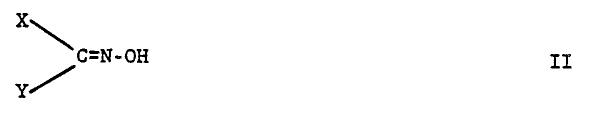 avec un méta-nitro diphényléther de formule III 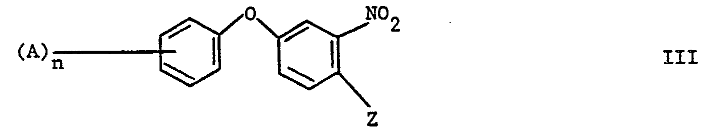 en présence d'une base forte.

6. Procédé selon la revendication 5, dans lequel la base forte est un hydrure de métal alcalin et la réaction est conduite dans un solvant organique aprotique polaire.

7. Procédé de lutte contre la croissance de plantes indésirables en un lieu, qui comprend le traitement du lieu par un composé tel que défini dans l'une quelconque des revendications 1 à 4 ou par une composition selon l'une quelconque des revendications 1 à 4.

8. L'utilisation d'un composé tel que défini dans l'une quelconque des revendications 1 à 4 comme herbicide.
